# EUROPEAN PATENT SPECIFICATION

(11) **EP 2 371 528 B1**
(45) Date of publication and mention of the grant of the patent: **13.10.2021**
(21) Application number: 09835009.3
(22) Date of filing: 25.12.2009
(51) Int. Cl.: B32B 5/02, A61K 8/02, A61K 8/72, A61K 8/73, A61K 8/84, A61L 15/00, D04H 1/72

(54) **METHOD FOR ATTACHING NANOFIBER SHEET**
VERFAHREN ZUR ANHAFTUNG VON NANOFASERFOLIEN UND NANOFASERFOLIE
PROCÉDÉ POUR FAIRE ADHÉRER UNE FEUILLE DE NANOFIBRES ET FEUILLE DE NANOFIBRES

(30) Priority: 26.12.2008 JP 2008334655
(43) Date of publication of application: 05.10.2011
(73) Proprietor: Kao Corporation, Chuo-ku Tokyo 103-8210 (JP)
(72) Inventor: TOJO, Takehiko, Haga-gun Tochigi 321-3497 (JP); ISHIKAWA, Masataka, Haga-gun Tochigi 321-3497 (JP)
(74) Representative: Vossius & Partner Patentanwälte Rechtsanwälte mbB
(86) International application number: PCT/JP2009/071560
(87) International publication number: WO 2010/074213

(56) References cited:
- WO-A1-2006/036130
- JP-A- 2008 162 098
- JP-A- 2008 190 093
- JP-T- 2008 514 341
- DATABASE WPI Week 200868 Thomson Scientific, London, GB; AN 2008-L60488 XP002678062, & JP 2008 179629 A (CS LAB KK) 7 August 2008 (2008-08-07)
- DATABASE WPI Week 200579 Thomson Scientific, London, GB; AN 2005-773311 XP002678063, & JP 2005 290610 A (NIPPON SUISAN KAISHA LTD) 20 October 2005 (2005-10-20)
- DATABASE WPI Week 200864 Thomson Scientific, London, GB; AN 2008-K76685 XP002678064, & JP 2008 162098 A (NIPPON SUISAN KAISHA LTD) 17 July 2008 (2008-07-17)
- None

## Description

### TECHNICAL FIELD

The present invention relates to a method for attaching a nanofiber sheet having a nanofiber layer made of nanofibers to the surface of an object.

### BACKGROUND ART

Nanofibers are applied to the fields demanding optical characteristics such as high transparency, where the nano-size effect of nanofibers are taken advantage of. For example, transparent fabric may be made of nanofibers with a diameter reduced to or below the wavelength of visible light. By the use of nanofibers the diameter of which is equal to the wavelength of visible light, structural color may be exhibited. Nanofibers also find applications in the fields demanding superabsorbent characteristics or high surface activity, where the super-specific surface area effect of nanofibers is taken advantage of, and in the fields demanding mechanical characteristics such as tensile strength and electrical characteristics such as high conductivity, where the supramolecular arrangement effect of nanofibers is made use of. Nanofibers having such characteristics have been used in the form of, for example, not only single fibers but aggregates (i.e., fabrics) or composites.

Embodiments of nanofibers that have been proposed include nanoscale polysaccharide fibers of 500 nm or smaller in diameter, made mainly of polysaccharides, and obtained by electrospinning (see patent literature 1 below). According to the disclosure of patent literature 1, the fibers are useful as a living tissue culture substratum in regenerative medicine or a part of a biomaterial, such as artificial valves, artificial organs, artificial vessels, and wound dressings, aiming at repair, regeneration, or treatment of living tissue deficiency.

A cosmetic sheet comprising a network structure made of a polymer nanofiber and a cosmetic or a cosmetic component held in the network structure has been proposed in patent literature 2 below. Patent Literature 2 alleges that the cosmetic sheet has not only improved adhesion or comfort to the user's face, hand, or leg but also preservability.

The general technical background of the invention also includes Patent Literature 3.

The sheets made of the nanofibers described in patent Literatures cited above have low stiffness due to the fineness of the nanofibers and cannot be said to have good handling properties. Therefore, it has not been easy to attach these sheets to the surface of an object, such as a human's face, for the purpose of obtaining the benefits, such as efficacy, expected of the sheet.

### Citation List

### Patent Literature

Patent Literature 1: JP 2005-290610A
Patent Literature 2: JP 2008-179629A
Patent Literature 3: WO2006/036130A

### SUMMARY OF INVENTION

The present invention provides a method of attaching a nanofiber sheet having a nanofiber layer including a polymeric nanofiber and a base layer located on one side of the nanofiber layer to a surface of an object, the method comprising bringing a side of the nanofiber layer of the nanofiber sheet into contact with the surface of the object under the condition that a surface of the nanofiber layer or the surface of the object is wet,wherein the base layer is releasable from the nanofiber layer, and the method further comprising removing the base layer from the nanofiber sheet that is attached to the surface of the object to transfer the nanofiber layer onto the surface of the object, and further comprising wetting the surface of the nanofiber layer or the surface of the object with a fluid having a probe tack of 3 to 2000 gf,wherein the probe tack is measured using a probe tack tester under conditions of a preload of 200 gf and a press time of 10 seconds.

The invention also provides nanofiber obtainable by the previously described method and including a nanofiber layer having a polymeric nanofiber and a base layer located on one side of the nanofiber layer, herein the base layer is an air-permeable sheet having an air resistance (Gurley) of 30 sec/100 ml or less.

### BRIEF DESCRIPTION OF DRAWINGS

[FIG. 1] FIG. 1 is a schematic cross-section showing a structure of an embodiment of the nanofiber sheet according to the invention.
[FIG. 2] FIG. 2 schematically shows a structure of a nanofiber.
[FIG. 3] FIG. 3 is a schematic view of an apparatus for carrying out electrospinning.

### DESCRIPTION OF EMBODIMENTS

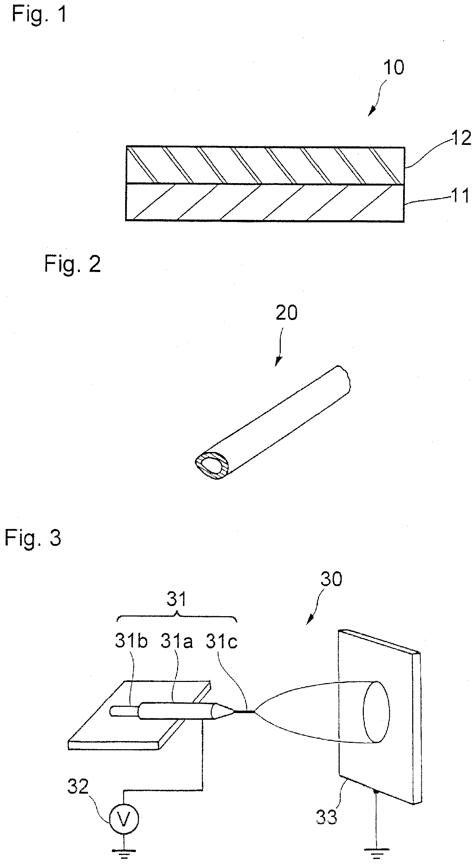

The invention will be described with reference to its preferred embodiments. Fig. 1 is a schematic cross-section illustrating an example of the structure of a nanofiber sheet used in the method of the invention. As illustrated, a nanofiber sheet 10 has a dual layer laminate structure. In detail, the nanofiber sheet 10 has a nanofiber layer 11 and a base layer 12 disposed on one side of the nanofiber layer 11. It is noted that Fig. 1 is merely illustrative and does not represent the actual thickness ratio of the layers.

The nanofiber layer 11 is made of a nanofiber. The nanofiber layer 11 may contain other components but preferably consists solely of a nanofiber. The nanofiber that can be used in the present embodiment usually has a thickness of 10 to 3000 nm, preferably 10 to 1000 nm, in terms of circle equivalent diameter. The thickness of nanofiber is measured by, for example, observation using a scanning electron microscope (SEM).

The length of the nanofiber is not critical and may have any length depending on the method of nanofiber manufacturing. In the nanofiber layer 11, the nanofiber may be disposed either unidirectionally or randomly. While the nanofiber is generally solid, a hollow nanofiber 20 as illustrated in Fig. 2 may be used as well.

The nanofiber comprises a polymeric material, either naturally occurring a or a synthetic. The polymeric material may be water soluble or insoluble. Examples of the naturally occurring polymeric materials include mucopolysaccharides, such as chitin, chitosan, hyaluronic acid, chondroitin sulfate, heparin, and keratosulfate; cellulose, pectin, xylan, lignin, glucomannan, galacturonic acid, psyllium seed gum, tamarind seed gum, gum arabic, tragacanth gum, modified corn starch, soybean water-soluble polysaccharides, alginic acid, carrageenan, laminaran, agar (agarose), and fucoidan.

Examples of the synthetic polymeric materials include polyvinyl alcohol, polystyrene, polycarbonate, polyacrylic acid, polymethyl acrylate, polyvinyl chloride, polyethylene terephthalate, polyamide 66, polyamide 46, polyurethane, polylactic acid, polycaprolactone, polyethylene glycol, polylactic glycolic acid, polyvinyl acetate, and polyethylene oxide.

The nanofiber layer 11 may have a thickness decided as appropriate to the intended use of the nanofiber sheet 10. For example, for use as attached to the human skin, the thickness of the nanofiber layer 11 is preferably 50 nm to 1 mm, more preferably 500 nm to 500 µm. The thickness of the nanofiber layer 11 may be determined using a contact thickness gauge Litematic VL-50A (from Mitutoyo Corp.) with a spherical carbide contact point of 5 mm in radius. A load of 0.01 Pa is applied to the sheet in the thickness measurement.

The nanofibers making up the nanofiber layer 11 are bonded to each other at the intersections thereof or entangled with each other, whereby the nanofiber layer 11 is self-supporting. Whether the nanofibers are bonded to or entangled with each other depends on the method of manufacture.

The base layer 12 on one side of the nanofiber layer 11 is used to improve handling properties of the nanofiber sheet 10. Specifically, the base layer 12 is provided to increase the stiffness of the nanofiber sheet 10. Used in combination with the base layer 12, the nanofiber layer 11 having low stiffness is attachable with good manageability to the surface of an object, such as human skin.

To make the nanofiber sheet 10 moderately stiff, it is preferred for the base layer 12 to have a Taber stiffness of 0.01 to 0.4 mNm, more preferably 0.01 to 0.2 mNm. Taber stiffness is determined in accordance with JIS P8125, "Determination of Stiffness".

Besides the Taber stiffness, the thickness of the base layer 12 is also influential on the handling properties of the nanofiber sheet 10. From this viewpoint, the thickness of the base layer 12 is preferably 5 to 500 µm, more preferably 10 to 300 µm, while varying with the material of the base layer 12. The thickness of the base layer 12 may be measured using a contact thickness gauge Litematic VL-50A. (from Mitutoyo Corp.).

The base layer 12 is provided directly on the nanofiber layer 11. The base layer 12 is releasably laminated to the nanofiber layer 11. The base layer 12 may be stripped off the nanofiber layer 11 after the nanofiber sheet 10 is attached on the side of the nanofiber layer 11 to, for example, human skin to leave only the nanofiber layer 11 on the human skin. Accordingly, it is preferred that there be no layers, such as a layer for adhesion, between the nanofiber layer 11 and the base layer 12.

The base layer 12 may be a film of synthetic resins, such as polyolefin resins and polyester resins. In the case where the base layer 12 is releasably laminated to the nanofiber layer 11, it is preferred that the side of the film facing the nanofiber layer 11 be previously subjected to a release treatment, such as application of a silicone resin or a corona discharge treatment, to have increased releasability. The thickness and Taber stiffness of the film preferably fall within the respective ranges recited above.

The base layer 12 is an air-permeable sheet. The base layer 12 is able to be releasably laminated to the nanofiber layer 11 with no particular need to perform a release treatment, such as application of a silicone resin. The air-permeable base layer 12 has an air resistance (Gurley) of 30 sec/100 ml or less, more preferably 20 sec/100 ml or less, as determined by the method specified in JIS P8117. Examples of air-permeable sheets are mesh sheets, fibrous sheets (e.g., woven, nonwoven, or knitted fabric and paper), and laminates thereof. Fibers that make the fibrous sheets may be fibers formed of fiber-forming synthetic resins or cellulosic natural fibers, such as cotton and pulp. The fibrous sheet preferably has a basis weight of 0.1 to 100 g/m², more preferably 0.5 to 50 g/m², in view of strength and handling properties. In using a mesh sheet as an air-permeable sheet, the mesh size is preferably 20 to 200 mesh/inch, more preferably 50 to 150 mesh/inch, as long as the air resistance is within the above described range. The wire diameter of the mesh is preferably 10 to 200 µm, more preferably 30 to 150 µm. The mesh sheet may be of any of the materials of the film described above.

As described, the nanofiber sheet 10 of the present embodiment has the nanofiber 11 exposed on one side thereof with the base layer 12 exposed on the other side. In attaching the nanofiber sheet 10 to the surface of an object, the surface of the nanofiber layer 11 is brought into contact with the surface to be stuck thereto.

Before the nanofiber sheet 10 is attached to the surface of an object, the surface of the object is wetted so that the nanofiber sheet 10 is successfully attached to the surface by action of the surface tension. The surface of the nanofiber layer 11 of the nanofiber sheet 10 may be wetted instead of wetting the surface of the object.

The surface of an object may be wetted by, for example, applying or spraying a fluid of various kinds to the surface. The fluid to be applied (or sprayed) is a substance containing a liquid component at the temperature at which the nanofiber sheet 10 is attached (hereinafter referred to as an attaching temperature) and having a viscosity of 5000 mPa·s or less at the attaching temperature. Such a fluid is exemplified by an aqueous liquid, such as an aqueous solution or dispersion, a non-aqueous solvent, and an aqueous solution or a dispersion of the non-aqueous solvent. Emulsions, including O/W emulsions and W/O emulsions, and liquids thickened with various thickeners, such as thickening polysaccharides, are also useful. More specifically, in the case when the nanofiber sheet 10 is used as, for example, a cosmetic mask, examples of the liquid wetting the surface of the object (skin) include skin lotion and beauty cream. The viscosity of the fluid is measured with a cone-plate viscometer.

In order to wet the surface of an object by applying or spraying a fluid, the inventors have revealed that it is only necessary that the fluid be applied in a minimum amount required for the fluid to sufficiently exhibit a surface tension. While the amount of a fluid to be applied varies with the size of the nanofiber sheet, the nanofiber sheet measuring, for example, 3 cm by 3 cm will easily be attached to the surface of an object in the presence of 0.01 cm³ of a fluid on the surface of an object.

Where the base layer 12 of the nanofiber sheet 10 is releasable from the nanofiber layer 11, attaching the nanofiber sheet 10 to the surface of an object may be followed by stripping the base layer 12 from the nanofiber sheet 10, thus transferring the nanofiber layer 11 onto the surface of the object.

To help smooth transfer of the nanofiber layer 11, the fluid to have a probe tack of 3 to 2000 gf, more preferably 5 to 1000 gf, at the attaching temperature. A fluid with such a probe tack is easy to apply to an object and spread over a necessary area and yet capable to provide a sufficiently higher adhesion between the object and the nanofiber layer 11 than that between the nanofiber layer 11 and the base layer 12, whereby only the nanofiber layer 11 is transferred to the object more manageably. Conversely, the adhesion between the nanofiber 11 and the object is not too strong to be removed from the object easily. From these considerations, it is preferred to use an aqueous solution of a thickening polysaccharide as a fluid for wetting as long as its probe tack falls within the range recited.

### Method of determining probe tack:

The probe tack determination is carried out in an environment of 23°C and 50% RH. A fluid to be tested (weighing about 0.01 to 0.1 g) is applied to a slide glass using a dropper and spread to a 2 cm diameter circle to make a film of the fluid. The probe tack is measured using a probe tack tester (from Rhesca Corp.) under conditions of a preload of 200 gf and a press time of 10 seconds.

The object to which the nanofiber sheet 10 is to be attached is chosen as appropriate to the material of the nanofiber, the material applied to the nanofibers, and the like. Examples of the object include human skin, skin and teeth of non-human mammals, and parts of plants, such as branches and foliage.

The nanofiber sheet 10 for use in the present embodiment is suitably produced by, for example, depositing a nanofiber on one side of a base layer 12 by electrospinning to form a nanofiber layer 11.

Fig. 3 illustrates an apparatus 30 for carrying out electrospinning. An apparatus 30 for achieving electrospinning includes a syringe 31, a high voltage supply 32, and a conductive collector 33. The syringe 31 has a cylinder 31a, a piston 31b, and a capillary 31c. The capillary 31c has an inner diameter of 10 to 1000 µm. The cylinder 31 a is filled with a solution of a polymer, the raw material of nanofibers. The solvent of the polymer solution is water or an organic solvent, which depends on the kind of the polymer. The high voltage supply 32 is, for example, a 10 to 30 kV direct voltage source. The positive pole of the high voltage supply 32 is electrically connected to the polymer solution in the syringe 31, with the negative pole grounded. The conductive collector 33 is, e.g., a metal plate that is grounded. The distance between the tip of the capillary 31c of the syringe 31 and the conductive collector 33 is set at, e.g., 30 to 300 mm. The apparatus 30 shown in Fig. 3 may be operated in the atmosphere. The operative environment is not particularly limited and may be, for example, 20° to 40°C and 10 to 50% RH.

With a voltage applied between the syringe 31 and the conductive collector 33, the piston 31b of the syringe 31 is slowly pressed inward to eject the polymer solution from the tip of the capillary 31c. As the jet travels toward the conductive collector 33, the solvent evaporates, and the polymer (solute) solidifies while being stretched due to the difference of electrical potential, thereby forming a nanofiber. By disposing an unshown sheet as a base layer on the surface of the conductive collector 33, the nanofiber is accumulated on the surface of the base layer. From the nature of the production process, the thus formed nanofiber is a continuous filament of infinite length. There is thus obtained a desired dual-layered nanofiber sheet 10. The hollow nanofiber as illustrated in Fig. 2 is obtained by, for example, using a double barreled capillary and feeding incompatible solutions in the core and the sheath.

While the invention has been described with reference to its preferred embodiments, it should be understood that the invention is not limited thereto. For example, while in the foregoing embodiments, the electrospinning technique is adopted to the production of nanofibers, the method for making nanofibers is not limited thereto.

While, according to the electrospinning technique shown in Fig. 3, the nanofiber formed is collected on the conductive collector 33 of plate shape, a conductive rotating drum may be used instead of the plate-shaped collector, in which case the nanofiber is deposited on the peripheral surface of the rotating drum.

As described, the invention allows for attaching a nanofiber sheet, which has not been very easy in handling, to the surface of an object with ease.

### Examples

The invention will now be illustrated in greater detail by way of Examples. It should be noted, however, that the invention is not construed as being limited thereto. Unless otherwise noted, all the percents are by weight.

### [Example 1] (not according to the invention)

A polylactic acid resin (L101 from Toray Industries, Inc.) was dissolved in a 80:20 (by weight) mixed solvent of chloroform and dimethylformamide to make a 9% solution. The solution was formed into a nanofiber using the electrospinning apparatus of Fig. 3 to form a nanofiber layer on the surface of a film as a base layer. The conditions of nanofiber production were as follows.
Applied voltage: 17 kV
Capillary-collector distance: 150 mm
Rate of ejection of solution: 1 ml/hr
Environment: 25°C, 30% RH

The film used as a base layer was a polyethylene terephthalate film (thickness: 25 µm; Taber stiffness: 0.08 mNm) having its one side treated with a silicone release agent. The nanofiber layer was formed on the release treated side. The thickness of the nanofiber layer formed was 30 µm. The thickness of the nanofiber was 300 nm.

Thus, a nanofiber sheet having the structure shown in Fig. 1 was obtained. The nanofiber sheet was attached on its nanofiber layer side to the skin of a human's upper arm that had previously been wetted with 0.75 g of water (probe tack (23°C): 0.75 g; viscosity (25°C, 10 rpm): 10 mPa·s or less) at 25°C. The base layer was then removed from the nanofiber layer, whereby the nanofiber layer was neatly transferred onto the skin.

### [Example 2] (not according to the invention)

A nanofiber sheet having the structure shown in Fig. 1 was obtained in the same manner as in Example 1, except for using a polyethylene terephthalate mesh sheet (mesh size: 120 mesh/inch; wire diameter: 63 µm) in place of the polyethylene terephthalate film. The mesh sheet had not been subjected to any release treatment. The mesh sheet had an air resistance of 0.1 sec/100 ml or less and a Taber stiffness of 0.13 mNm. The same procedure for transfer as in Example 1 was followed using the resulting nanofiber sheet, whereby the nanofiber layer was successfully transferred to the human skin.

### [Example 3]

A nanofiber sheet having the structure shown in Fig. 1 was obtained in the same manner as in Example 1, except for using paper for plain paper copiers (basis weight: 78 g/m²; thickness: 0.09 mm) in place of the polyethylene terephthalate film. The paper had an air resistance of 21 seconds 100 ml and a Taber stiffness of 0.12 mNm. The operation of transfer was carried out in the same manner as in Example 1, except for replacing water with a 15% aqueous solution of pullulan (probe tack (23°C): 59.9 g; viscosity (25°C, 10 rpm): 560 mPa·s) to wet the skin of a human upper arm. As a result, the nanofiber layer was transferred to the human skin more successfully than in Example 1.

### [Example 4]

The operation of transfer was carried out in the same manner as in Example 3, except for replacing the 15% aqueous solution of pullulan with a 10% aqueous solution of pullulan (probe tack (23°C): 10.7 g; viscosity (25°C, 10 rpm): 123 mPa·s) to wet the skin of a human upper arm. As a result, the nanofiber layer was transferred to the human skin more successfully than in Example 1.

### [Example 5]

The operation of transfer was carried out in the same manner as in Example 3, except for replacing the 15% aqueous solution of pullulan with a 30% aqueous solution of pullulan (probe tack (23°C): 306.5 g) to wet the skin of a human upper arm. As a result, the nanofiber layer was transferred to the human skin more successfully than in Example 1.

### [Example 6]

The operation of transfer was carried out in the same manner as in Example 3, except for replacing the 15% aqueous solution of pullulan with a urethane aqueous solution (DynamX, from Akzonobel; probe tack (23°C): 868 g) to wet the skin of a human upper arm. As a result, the nanofiber layer was transferred to the human skin more successfully than in Example 1.

### [Example 7]

The operation of transfer was carried out in the same manner as in Example 3, except for replacing the 15% aqueous solution of pullulan with a trimethylsiloxysilicic acid aqueous solution (KF-9021, from Shin-Etsu Chemical; probe tack (23°C): 1862 g) to wet the skin of a human upper arm. As a result, the nanofiber layer was transferred to the human skin successfully.

### [Example 8]

The operation of transfer was carried out in the same manner as in Example 3, except for replacing the 15% aqueous solution of pullulan with a skin lotion (Sofina Wrinkle Seraty Essence, from Kao Corp.; probe tack (23°C): 8.6 g; viscosity (25°C, 10 rpm): 600 mPa·s) to wet the skin of a human upper arm. As a result, the nanofiber layer was transferred to the human skin more successfully than in Example 1.

### [Example 9]

A nanofiber sheet having the structure shown in Fig. 1 was obtained in the same manner as in Example 1, except for using a spun-bonded nonwoven fabric (basis weight: 40 g/m2; thickness: 0.28 mm) in place of the polyethylene terephthalate film. The nonwoven fabric was of sheath/core conjugate fiber composed of polyethylene terephthalate as a core and polyethylene as a sheath. The nonwoven fabric had an air resistance of 0.1 sec/100 ml and a Taber stiffness of 0.06 mNm. The operation of transfer was carried out using the resulting nanofiber sheet in the same manner as in Example 1, except for replacing water with a 15% aqueous solution of pullulan (probe tack (23°C): 59.9 g; viscosity (25°C, 10 rpm): 560 mPa·s) to wet the skin of a human upper arm. As a result, the nanofiber layer was transferred to the human skin more successfully than in Example 1.

## Claims

1. A method of attaching a nanofiber sheet having a nanofiber layer comprising a polymeric nanofiber and a base layer located on one side of the nanofiber layer to a surface of an object, the method comprising bringing a side of the nanofiber layer of the nanofiber sheet into contact with the surface of the object under the condition that a surface of the nanofiber layer or the surface of the object is wet, wherein the base layer is releasable from the nanofiber layer, and the method further comprising removing the base layer from the nanofiber sheet that is attached to the surface of the object to transfer the nanofiber layer onto the surface of the object and further comprising wetting the surface of the nanofiber layer or the surface of the object with a fluid having a probe tack of 3 to 2000 gf, wherein the probe tack is measured using a probe tack tester under conditions of a preload of 200 gf and a press time of 10 seconds.

2. The method according to claim 1, wherein the base layer has a Taber stiffness of 0.01 to 0.4 mNm determined in accordance with JIS P8125.

3. The method according to claim 1 or 2, wherein the nanofiber has a diameter of 10 to 1000 nm.

4. The method according to any one of claims 1 to 3, wherein the base layer comprises an air-permeable sheet having an air resistance by Gurley method of 30 sec/100 ml or less.

5. The method according to claim 4 wherein the air-permeable sheet is a mesh sheet, a fibrous sheet or a laminate thereof.

6. The method according to claim 5, wherein the fibrous sheet has a basis weight of 0.1 to 100 g/m².

7. The method according to claim 5, wherein the mesh size of the mesh sheet is 20 to 200 mesh/inch.

8. A nanofiber obtainable by the method of claim 1 and comprising a nanofiber layer having a polymeric nanofiber and a base layer located on one side of the nanofiber layer, wherein the base layer is an air-permeable sheet having an air resistance (Gurley) of 30 sec/100 ml or less as determined by the method specified in JIS P8117.

## Patentansprüche

1. Ein Verfahren zur Befestigung einer Nanofaserfolie mit einer Nanofaserschicht, umfassend eine polymere Nanofaser und eine Basisschicht, die sich auf einer Seite der Nanofaserschicht befindet, an eine Oberfläche eines Objekts, wobei das Verfahren das In-Kontakt-Bringen einer Seite der Nanofaserschicht der Nanofaserfolie mit der Oberfläche des Objekts unter der Bedingung, dass eine Oberfläche der Nanofaserschicht oder die Oberfläche des Objekts feucht ist, umfasst, wobei die Basisschicht von der Nanofaserschicht ablösbar ist, und wobei das Verfahren ferner das Entfernen der Basisschicht von der Nanofaserfolie, die an der Oberfläche des Objekts befestigt ist, umfasst, um die Nanofaserschicht auf die Oberfläche des Objekts zu übertragen, und ferner das Benetzen der Oberfläche der Nanofaserschicht oder der Oberfläche des Objekts mit einem Fluid mit einer Prüfklebrigkeit von 3 bis 2000 gf, wobei die Prüfklebrigkeit mit einem Probe-Tack-Tester unter den Bedingungen einer Vorbelastung von 200 gf und einer Presszeit von 10 Sekunden gemessen wird.

2. Das Verfahren nach Anspruch 1, wobei die Basisschicht eine Taber-Steifigkeit von 0,01 bis 0,4 mNm, bestimmt nach JIS P8125, aufweist.

3. Das Verfahren nach Anspruch 1 oder 2, wobei die Nanofaser einen Durchmesser von 10 bis 1000 nm aufweist.

4. Das Verfahren nach einem der Ansprüche 1 bis 3, wobei die Basisschicht eine luftdurchlässige Folie mit einem Luftwiderstand nach dem Gurley-Verfahren von 30 s/100 ml oder weniger umfasst.

5. Das Verfahren nach Anspruch 4, wobei die luftdurchlässige Folie eine Maschenfolie, eine Faserfolie oder ein Laminat daraus ist.

6. Das Verfahren nach Anspruch 5, wobei die Faserfolie ein Basisgewicht von 0,1 bis 100 g/m² aufweist.

7. Das Verfahren nach Anspruch 5, wobei die Maschengröße der Maschenfolie 20 bis 200 mesh/inch beträgt.

8. Eine Nanofaser, die nach dem Verfahren nach Anspruch 1 erhältlich ist und eine Nanofaserschicht mit einer polymeren Nanofaser und eine Basisschicht, die sich auf einer Seite der Nanofaserschicht befindet, umfasst, wobei die Basisschicht eine luftdurchlässige Folie mit einem Luftwiderstand (Gurley) von 30 s/100 ml oder weniger ist, wie durch das in JIS P8117 angegebene Verfahren bestimmt.

## Revendications

1. Procédé pour attacher une feuille de nanofibres ayant une couche de nanofibres comprenant des nanofibres polymères et une couche de base située sur un côté de la couche de nanofibres à une surface d'un objet, le procédé comprenant la mise en contact d'un côté de la couche de nanofibres de la feuille de nanofibres avec la surface de l'objet dans des conditions dans lesquelles une surface de la couche de nanofibres ou la surface de l'objet est humide, dans lequel la couche de base est détachable de la couche de nanofibres, le procédé comprenant en outre le retrait de la couche de base à partir de la feuille de nanofibres qui est attachée à la surface de l'objet pour transférer la couche de nanofibres sur la surface de l'objet, et comprenant en outre le mouillage de la surface de la couche de nanofibres ou de la surface de l'objet avec un fluide ayant une pégosité à la sonde de 3 à 2 000 gf, dans lequel la pégosité à la sonde est mesurée par utilisation d'un testeur de pégosité à la sonde dans des conditions de précharge de 200 gf et de temps de pressage de 10 secondes.

2. Procédé selon la revendication 1, dans lequel la couche de base a une rigidité Taber de 0,01 à 0,4 mNm, déterminée conformément à la norme JIS P8125.

3. Procédé selon la revendication 1 ou 2, dans lequel les nanofibres ont un diamètre de 10 à 1 000 nm.

4. Procédé selon l'une quelconque des revendications 1 à 3, dans lequel la couche de base comprend une feuille perméable à l'air ayant une résistance à l'air d'après la méthode Gurley de 30 s/100 ml ou moins.

5. Procédé selon la revendication 4, dans lequel la feuille perméable à l'air est une feuille maillée, une feuille fibreuse ou un stratifié de celles-ci.

6. Procédé selon la revendication 5, dans lequel la feuille fibreuse a une masse surfacique de 0,1 à 100 g/m².

7. Procédé selon la revendication 5, dans lequel la taille de maillage de la feuille maillée est de 20 à 200 mailles/pouce.

8. Nanofibre susceptible d'être obtenue par le procédé de la revendication 1 et comprenant une couche de nanofibres ayant des nanofibres polymères et une couche de base située sur un côté de la couche de nanofibres, dans laquelle la couche de base est une feuille perméable à l'air ayant une résistance à l'air (Gurley) de 30 s/100 ml ou moins, telle que déterminée par la méthode spécifiée dans la norme JIS P8117.
